# EUROPÄISCHE PATENTSCHRIFT

(11) **EP 0 616 494 B1**
(45) Veröffentlichungstag und Bekanntmachung des Hinweises auf die Patenterteilung: **30.09.1998**
(21) Anmeldenummer: 92917460.5
(22) Anmeldetag: 23.05.1992
(51) Int. Cl.: A01N 43/40

(54) **1- N-(HALO-3-PYRIDYLMETHYL)]-N-METHYLAMINO-1-ALKYLAMINO-2-NITROETHYLEN-DERIVATE ZUR BEKÄMPFUNG VON FLÖHEN BEI HAUSTIEREN**
1- N-(HALO-3-PYRIDYLMETHYL)]-N-METHYLAMINO-1-ALKYLAMINO-2-NITROETHYLENE DERIVATIVES FOR USE AGAINST FLEAS ON PETS
DERIVES DE 1- N-(HALO-3-PYRIDYLMETHYLE)]-N-METHYLAMINO-1-ALKYLAMINO-2-NITROETHYLENE POUR LUTTER CONTRE LES PUCES CHEZ LES ANIMAUX DOMESTIQUES

(43) Veröffentlichungstag der Anmeldung: 28.09.1994
(73) Patentinhaber: Novartis AG, 4058 Basel (CH)
(72) Erfinder: FRIEDEL, Thomas, East Blaxland, NSW 2774 (AU); MOYSES, Eric, William, CH-4058 Basel (CH); TINEMBART, Olivier, CH-2800 Delémont (CH); MAIENFISCH, Peter, CH-4118 Rodersdorf (CH); GSELL, Laurenz, CH-4056 Basel (CH)
(86) Internationale Anmeldenummer: EP9201161
(87) Internationale Veröffentlichungsnummer: WO9324002

(56) Entgegenhaltungen:
- EP-A- 0 255 803
- EP-A- 0 302 389
- EP-A- 0 302 833
- EP-A- 0 375 907

## Beschreibung

Die vorliegende Erfindung betrifft 1-[N-(Halo-3-pyridylmethyl)]-N-methylamino-1-alkylamino-2-nitroethylen-Derivate der nachstehenden Formel I zur Verwendung in einem Verfahren zur Bekämpfung von Flöhen bei Haustieren, insbesondere bei Hunden und Katzen, wobei die systemische Applikation bevorzugt ist. Sie betrifft auch ein Verfahren zur Eindämmung eines Flohbefalls an Hunden und Katzen, das dadurch charakterisiert ist, dass man eine gegen Flöhe wirksame Menge der genannten Substanz über den Verdauungstrakt oder über das Blut des Wirtstieres an das besagte Haustier, z.B. den Hund oder die Katze systemisch verabreicht.

Die eingangs genannten 1-[N-(Halo-3-pyridylmethyl)]-N-methylamino-1-alkylamino-2-nitroethylen-Derivate haben folgende chemische Struktur der Formel I: 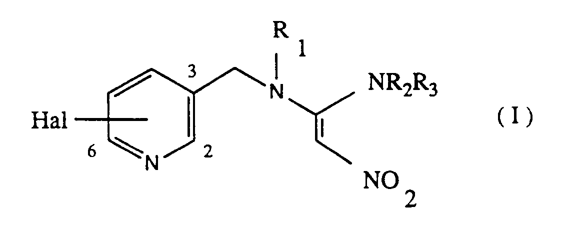 worin
Hal für Halogen, wie Fluor, Chlor, Brom oder Jod steht;
R₁ Wasserstoff, C₁-C₆-Alkyl oder C₃-C₇-Cycloalkyl bedeutet;
R₂ für Wasserstoff, C₁-C₆-Alkyl oder C₃-C₇-Cycloalkyl steht und
R₃ für Wasserstoff oder C₁-C₆-Alkyl steht.

Bevorzugt im Rahmen der Formel I sind wegen ihrer ausgeprägten Antiflohwirkung folgende Untergruppen:
Gruppe a: Verbindungen der Formel I, worin Hal in der 6-Position steht. Besonders bevorzugt sind dabei jene Vertreter worin Hal für Fluor, Chlor oder Brom steht, insbesondere die Chlor-Vertreter.
Gruppe b: Verbindungen der Formel I, worin R₁ für Wasserstoff, C₁-C₃-Alkyl oder C₃-C₆-Cycloalkyl , vorzugsweise für Wasserstoff, Methyl oder Ethyl oder Cyclopropyl, insbesondere für Ethyl steht.
Gruppe c: Verbindungen der Formel I, worin R₂ für C₁-C₃-Alkyl oder Cyclopropyl, insbesondere für Methyl steht.
Gruppe d: Verbindungen der Formel I, worin Hal für Halogen, wie Fluor, Chlor, Brom oder Jod steht; R₁ Wasserstoff, Methyl, C₃--C₆-Alkyl oder C₃-C₇-Cycloalkyl bedeutet und R₂ für Wasserstoff oder C₁-C₆-Alkyl steht.

Innnerhalb der Gruppen (a) bis (d) sind vor allem jene Vertreter bevorzugt, bei denen R₃ für Wasserstoff steht.

Besonders bevorzugt wegen ihrer ausgeprägten systemischen Antiflohaktivität ist die Verbindung:
1-[N-(6-Chlor-3-pyridylmethyl)]-N-ethylamino-1-methylamino-2-nitroethylen, aber auch ihre unmittelbaren Homologen.

Unter dem Begriff Alkyl sind im Rahmen vorliegender Erfindung je nach Anzahl der angegebenen Kohlenstoffatome folgende geradkettige und verzweigte Gruppen zu verstehen, z.B.: Methyl, Ethyl, n-Propyl, iso-Propyl, n-Butyl, sek.-Butyl, tert.Butyl, Isobutyl, etc.. Unter Hal soll hier und im folgenden Fluor, Chlor, Brom oder Jod, vorzugsweise Fluor, Chlor oder Brom, vor allem jedoch Chlor verstanden werden. Cycloalkyl selbst oder als Bestandteil eines Substituenten steht je nach Anzahl der angegebenen Kohlenstoffatome für Cyclopropyl, Cyclobutyl, Cyclopentyl, Cyclohexyl, etc., wobei Cyclopropyl besonders bevorzugt ist.

Verbindungen des unter Formel I fallenden Strukturtypus werden einschliesslich ihres Herstellungsverfahrens in EP-0'302'389 beschrieben. Die Verbindungen der Formel I können nach den EP-0'302'389 offenbarten Methoden oder analog zu den dort beschriebenen Substanzen hergestellt werden. Diese Publikation offenbart eine weit gefasste Klasse von Substanzen, von denen die obigen Vertreter nur eine ausgewählte Untergruppe darstellen, die dort jedoch nicht als Gruppe in Erscheinung tritt. Die in EP-0'302'389 offenbarte Substanzklasse wird als insektizid und akarizid beschreiben. Als bevorzugtes Anwendungsgebiet werden pflanzenschädigende Insekten und Milben angegeben. Ein Hinweis auf eine Antiflohwirkung fehlt.

EP-0'302'833 offenbart ebenfalls eine Gruppe von Substanzen, die strukturell denjenigen der obigen Formel I nahekommen oder zum Teil strukturell mit diesen überlappen. Der wesentliche strukturelle Unterschied der konkret offenbarten Substanzen gegenüber denen der Formel I besteht darin, dass sie eine unsubstituierte Pyridylgruppe aufweisen, wahrend die Verbindungen der Formel I am Pyridylrest einfach halogeniert sind. Von den in EP-0'302'833 offenbarten Verbindungen wird gesagt, dass sie bei günstiger Warmblüter- und Pflanzenverträglichkeit wertvolle Wirkstoffe in der Schädlingsbekämpfung darstellen und sich zur Bekämpfung von Schädlingen an Tieren und Pflanzen eignen. Unter den tierschädigenden Insekten wird die Ordnung Siphonaptera (Flöhe) spezifisch genannt.

In EP-0'255'803 werden ausserdem ausschliesslich die drei ovizid und larvizid wirksamen Wirkstoffklassen Juvenilhormone, Benzoylharnstoffe und Triazinderivate zur Lösung des Problems offenbart.

Es wurde nun überraschenderweise gefunden, dass bei den Verbindungen der obigen Formel I durch Eiführung eines Halogenatoms in den Pyridylrest eine ganz unerwartete und signifikant gesteigerte Antiflohaktivität auftritt. Dies geht eindeutig aus dem Vergleich des erfindungsgemässen Grundvertreters und des strukturell nächstvergleichbaren Vertreters des Standes der Technik hervor. Die entsprechenden Vergleichsdaten sind weiter unten in den biologischen Beispielen angegeben.

Die hervorragende Aktivität der Verbindungen der Formel I gegen Flöhe ist deshalb so bedeutungsvoll, weil der Befall von Flöhen an Haus- und Schosstieren, insbesondere bei Hunden und Katzen immer noch ein nur unzureichend gelöstes Problem für den Veterinär und den Tierhalter darstellt.

Auf Grund des komplizierten Lebenszykluses von Flöhen ist keine der bekannten Methoden zur Bekämpfung dieser überaus lästigen Parasiten, die nicht nur Krankheiten übertragen, sondern auch unangenehme Allergien verursachen, rundum zufriedenstellend, insbesondere, weil die meisten der bekannten Bekämpfungsmethoden darauf beruhen, dass die Aktivsubstanz auf den Lebensraum der verschiedenen Flohstadien appliziert wird. Diese Vorgehensweise ist jedoch wegen des komplexen Lebenszyklus der Flöhe sehr aufwendig, kann faktisch nicht alle Lebensräume erfassen und ist daher entsprechend unzuverlässig.
Ist die Bekämpfung z.B. auf die Behandlung der ausgewachsenen Flöhe im Fell ausgerichtet, was üblicherweise durch Applikation eines Antiflohmittels auf das Fell des Wirtstieres bewerkstelligt wird, so bleiben die unterschiedlichen juvenilen Stadien der Flöhe, die nicht nur im Fell des Tieres, sondern hauptsächlich auf dem Boden, auf Teppichen, auf dem Schlafplatz des Tieres, auf Stühlen, im Garten und all den andern Plätzen, mit denen das befallene Tier in Berührung kommt, existieren, völlig ausser acht. Erwachsene Katzen- und Hundeflöhe (Ctenocephalides felis und C. canis) leben normalerweise im Fell der Wirtskatze bzw. des Wirtshundes. Sie ernähren sich vom Blut des Wirtstieres und legen ihre Eier in sein Fell. Da diese Eier jedoch nicht selbsthaftend sind, fallen sie im allgemeinen rasch ab und können am Boden, auf Teppichen, im Hunde- und Katzenkorb, auf den vom Tier benutzten Stühlen, im Garten, im Hinterhof, usw. gefunden werden.

Das bedeutet, dass der gesamte Lebensraum der Schosstiere mit Floheiern verseucht ist, aus denen sich innerhalb von zwei Tagen die Larven bilden. Man unterscheidet bei den Larven drei Entwicklungsstadien, die jeweils drei Tage dauern. Im letzen Stadium spinnt die Larve ihren Kokon und verwandelt sich in die Puppe. Unter günstigen Bedingungen, d.h. 33°C und einer relativen Luftfeuchtigkeit von 65 %, findet die Umwandlung vom Ei zur Puppe in etwa 8 bis 10 Tagen statt. Nach etwa weiteren 8 Tagen entstehen in den noch immer auf dem Boden, den Teppichen, den Schlafplätzen, den Stühlen, usw. liegenden Kokons die jungen, fertigen Flöhe. Die jungen erwachsenen Flöhe verharren dort bis sie die Gegenwart eines akzeptablen Wirtstieres spüren dann schlüpfen sie aus ihrem Kokon und versuchen auf das Wirtstier zu springen. Daraus ersieht man, dass es mindestens drei Wochen dauert, bis sich aus einem Ei ein erwachsener Jungfloh entwickelt, der in der Lage ist, das Wirtstier erneut zu befallen.

Dieser Jungfloh kann jedoch monatelang, möglicherweise bis zu einem Jahr in seinem Kokon verbleiben. Andererseits kann unter weniger optimalen Bedingungen die Entwicklung vom Ei bis zum erwachsenen Jungfloh 4 bis 5 Monate beanspruchen. Flöhe benötigen zur Erlangung ihrer sexuellen Reife Blut als Nahrungsmittel, um sich vermehren zu können, und dieses Blut muss auch vom richtigen Wirtstier stammen. Es wird normalerweise den Ausscheidungen der adulten Flöhe entnommen, die auf dem Wirtstier leben. Diese Ausscheidungen enthalten hohe Anteile an unverdautem Blut.

Dieser lange Lebenszyklus, der getrennt vom Wirtstier stattfindet, hat einen bedeutenden Einfluss auf eine erfolgreiche Flohbekämpfung am Wirtstier.

Nur wenn sich die Flöhe im Fell des Wirtstieres sehr rasch erfolgreich bekämpfen lassen, d.h., wenn alle erwachsenen Flöhe in sehr kurzer Zeit durch eine entsprechende Wirksubstanz abgetötet werden, ist die Katze bzw. der Hund vor dem Risiko eines Wiederbefalls durch neu ausgeschlüpfte Jungflöhe aus seinem Lebensraum bewahrt.

Der Flohbefall von Hunden und Katzen hat nicht nur für das zu behandelnde Tier, sondern auch für den Tierhalter unangenehme Begleiterscheinungen. Derartige Unannehmlichkeiten führen z.B. zu lokalen Reizungen oder lästigem Juckreiz und münden oft zu heftigem Kratzen. Ein grosser Teil der Tiere wird allergisch gegen die Ausscheidungen der Flöhe, was auf dem Tierkörper zu sehr juckenden und krustigen Hautveränderungen rund um die Biss-Stellen führt. Diese Hautveränderungen weisen normalerweise einen Durchmesser von etwa 3 mm oder mehr auf und machen das Tier oft bissig und veranlassen es zu kratzen, so dass es in der Folge stellenweise zu Haarausfall kommt.

Darüberhinaus sind flohinfizierte Tiere dauernd der Gefahr ausgesetzt, dass sie sich mit Dipylidium, einer Bandwurmart, infizieren, die durch Flöhe übertragen wird.

Der Flohbefall ist nicht nur für das befallene Tier äusserst lästig, sondern verursacht auch bei dem Tierhalter unangenehme Begleiterscheinungen, dann schliesslich erkennt dieser an dem ungewöhnlichen Verhalten seines Schosstieres, dass dieses krank ist und leidet und, dass er ihm helfen muss. Darüberhinaus kann es für den Tierhalter unangenehm werden, wenn er sein befallenes Tier abschafft, dieses stirbt oder es zeitweise aus der gewohnten Umgebung entfernt wird, da die auf dem Boden befindlichen neu entpuppten Flöhe bei langer Abwesenheit eines geeigneten Wirtstieres notgedrungenermassen den Menschen befallen, obwohl sie sich mit menschlichem Blut als einziger Nahrungsquelle nicht vermehren können. Selbst wenn der Hund oder die Katze anwesend ist, kann der Tierhalter von den Flöhen gebissen werden.

Ferner können Hunde- und Katzenflöhe, bzw. deren Ausscheidungen bei manchen Menschen zu Allergie-ähnlichen Hauterkrankungen führen, die in manchen Fällen zur Abschaffung des Schosstieres zwingen. Eine wirksame Bekämpfung der Flöhe bei Hunden und Katzen war daher schon von jeher wünschenswert.

Es sind eine Reihe herkömmlicher Bekämpfungsmethoden bekannt, die jedoch verschiedenartige Nachteile aufweisen. Benützt man z.B. Flohkämme, die oberflächlich mit einem Insektizid kodiert sind, so bleibt dem Tierhalter nichts anderes übrig, als das Tier intensiv und oft zu kämmen, was je nach Grösse des Tieres wenige Minuten bis zu einer Stunde in Anspruch nehmen kann und nicht von jedem Tier geduldig hingenommen wird. Aber auch nicht jeder Tierhalter ist bereit die Zeit hierfür aufzubringen. Die Verwendung von entsprechenden Floh-aktiven Shampoos kann in vielen Fällen nicht erfolgen, da die meisten Katzen aber auch zahlreiche Hunde sich nicht oder nur unter Gewaltanwendung baden lassen, so das Wasser und Wirkstoff verspritzt werden, die beseitigt werden müsssen. Ausserdem hält die Wirkung einer solchen Bade-Behandlung höchstens ca. eine Woche lang an, und die umständliche Prozedur muss wiederholt werden. Mit den selben oder ganz ähnlichen Problemen hat man bei der Verwendung von Einölungen (dips) oder Spülungen (rinses) zu rechnen. Auch der Einsatz von Pudern (dusting powders) wird vom Tier im allgemeinen nicht widerstandslos hingenommen, da man doch einige Minuten braucht, und die gesamte Felloberfläche gleichmässig zu behandeln, wobei zwangsläufig etwas Staub in Maul, Nase und Augen gelangt. Selbst bei sorgfältiger Anwendung kann nicht ausgeschlossen werden, dass Tier und Mensch von dem Puder inhalieren. Es ist praktisch unvermeidbar, dass auch der Mensch in mehr oder weniger intensiven Kontakt mit dem Mittel kommt.

Bei dem Einsatz von Sprays kann manch einer die unliebsame Ueberraschung erleben, da die meisten Tiere insbesondere Katzen, bereits bei dem Sprühgerausch die Flucht ergreifen oder aggressiv reagieren. Darüberhinaus haben Sprays auch alle unter den Pudern aufgezählten Nachteile, wobei hinzukommt, dass sie sich noch feiner in der Atmosphäre verteilen und daher von Mensch und Tier inhaliert werden. Flöhe werden häufig auch mit sogenannten Floh-Halsbändern bekämpft, die vorübergehend eine gute Effektivität gewährleisten. Eine gewisse Schwäche zeigt sich bei dieser Behandlung insbesondere durch die lokal sehr begrenzte Applikation. Zwar beträgt die abtötende Wirkung im Hals- und Brustbereich im allgemeinen 100 %; weiter wegliegende Körperpartien wurden jedoch kaum beeinflusst. Ausserdem ist die Wirkung dieser Bänder zeitlich begrenzt. Daneben sehen viele dieser Halsbänder unattraktiv aus und können das Tier stören. Man kann heute auch Medaillons kaufen, die an gewöhnliche Halsbänder gehängt werden können und aktiv sein sollen. Diese sehen zwar optisch ansprechend aus, ihre Wirkung ist jedoch unbefriedigend, da der Fellkontakt mangelhaft ist. Einige floh-aktive Organophosphorverbindungen werden auch als Spot-On-Formulierungen angeboten und somit auf einer lokal begrenzten Stelle des Fells aufgetragen. Sie zeigen im allgemeinen gegen adulte Flöhe kurzfristig gute Wirkung, wobei die angewendeten Mittel oft jedoch problematische Toxwerte aufweisen. Organophosphorverbindungen wurden zum Teil auch oral verabreicht, wobei ihnen jedoch enge Sicherheitsgrenzen gesetzt sind und die keinesfalls mit anderen Organophosphorverbindungen gleichzeitig appliziert werden dürfen.

Insgesamt kann gesagt werden, dass die bisherigen Verfahren, die die Abtötung des adulten Flohs anstreben, vor allem deshalb so unbefridigende Ergebnisse liefern, weil sie von der Geduld und der Geschicklichkeit des Anwenders mit dem befallenen Wirtstieres umzugehen abhängen. Der Erfolg der gängigen Mittel steht und fällt damit, wie oft und wie umfassend der Anwender, und das ist im Normalfall der Tierhalter, den Wirkstoff auf das Wirtstier appliziert und wie gründlich er die Umgebung, in der das Wirtstier lebt, desinfiziert. Die bisherigen Methoden sind relativ aufwendig, zeitraubend und langfristig nicht besonders erfolgsversprechend. Kurzfristig lassen sich mit den herkömmlichen Mitteln durchaus Linderungen erreichen.
Was bei den herkömmlichen Methoden bzw. Mitteln bisher nicht genügend beachtet wurde, ist die Tatsache, dass auf Grund des besonderen Lebenszyklus der Flöhe Hunde und Katzen immer wieder von neuen infiziert werden, zum einen, da ein Kontakt mit den Floheiern, Flohlarven und jungen, adulten Flöhen auf dem Boden bzw. in der nächsten Umgebung des Tieres micht vermeidbar ist, zum anderen, weil viele Schosstiere immer wieder mit infizierten Artgenossen in Berührung kommen.

Die ständig wiederkehrende Reinfestation wird mit herkömmlichen Mitteln unzureichend verhindert oder ist nur mit grossen Aufwandmengen an Desinfektionsmittel etc. erreichbar.

Es wurde nun überraschenderweise festgestellt, dass man mit Hilfe bestimmter systemischer Applikationsarten und unter Einsatz der Verbindungen der Formel I als Aktivsubstanzen, die die adulten Flöhe sehr rasch und vollständig beseitigen kann und dadurch blockierend in den komplexen Entwicklungszyklus der Flöhe eingreifen kann. Da diese Substanzen ihre ausgezeichnete Antiflohwirkung auch dann noch voll entfaltet, wenn man sie systemisch, d.h. oral, parenteral, subkutan, intramuskulär oder intravenös an das Wirtstier verabreicht, ist es möglich, durch ihre gezielte periodische Verabreichung den geschilderten dargestellten Teufelskreis der ständig wiederkehrenden Reinfestation auf einfache Weise durchbrechen, bis alle Jungstadien im Lebensbereich des Wirtstieres ausgestorben sind. Die Flöhe werden getötet, an ihrer Vermehrung gehindert, die juvenilen Stadien werden am Heranwachsen gehindert, können das Wirtstier nicht mehr befallen, wodurch der Lebensraum von Hunden und Katzen auf Dauer flohfrei gehalten werden kann. Die einzige unvermeidbare Reinfestationsmöglichkeit erstreckt sich auf den Kontakt mit befallenen Artgenossen und dieser Aspekt kann naturgemäss nur mit Hilfe einer Dauerbehandlung hundertprozentig ausgeschlossen werden. Dieses Restrisiko ist jedoch von untergeordneter Bedeutung.

Es wurde nunmehr gefunden, dass durch orale Gabe, einer gegen Flöhe wirksamen Menge von Verbindungen der Formel I der Befall durch Flöhe an Haustieren, wie Katzen und Hunden drastisch reduziert oder völlig verhindert werden kann.

Erstaunlich im Zusammenhang mit der vorliegenden Erfindung ist jedoch, dass die volle Wirkung auch dann noch erreicht wird, wenn man einen der Wirkstoff in relativ niedrigen Aufwandmengen an das Wirtstier verabreicht und er erst auf dem Umweg über den Magendarmtrakt und somit über das aufgesaugte Blut den adulten Floh als Zielobjekt erreicht. Mit der vollständigen Abtötung der adulten Flöhe nach systemischer Verabreichung der Wirksubstanz ist nunmehr eine Ausmerzung der Flöhe errreichbar. Durch Kombination dieser systemischen Anwendung der Wirksubstanz mit begleitenden Massnahmen, z.B. Desinfektion des Aufenthaltsortes des Wirtstieres ist eine noch rasche Beseitigung des Flohproblems möglich; aber auch ohne diese begleitenden Massnahmen wird die Flohpopulation innerhalb wenigen Wochen oder spätestens nach Monaten ganz oder auf ein akzeptables Minimum reduziert.

Die Verbindungen der Formel I zeigen insofern eine Wirkung auf juvenile Flohstadien, dadurch, dass Flohlarven, die aus den Floheiern schlüpfen, im wesentlichen auf die Ausscheidungen der erwachsenen Flöhe angewiesen sind, da sie sich von diesen Exkrementen ernähren. Die Ausscheidungen von Flöhen enthalten jedoch noch hohe Anteile an unverdautem Blut des Wirtstieres und dienen den sich entwickelnden Flöhen als Proteinquelle. Da die Wirksubstanzen der Formel I die adulten Flöhe jedoch sehr rasch abtötet, fehlen die notwendigen Ausscheidungen, und den Jungstadien ist der Nährboden entzogen, so dass diese zugrunde gehen bevor sie das Adultenstadium erreichen. Auch dies trägt massgeblich zur Unterbrechung des komplexen Lebenszyklus der Flöhe bei und verhindert, dass die Wirtstiere sich in ihrem bevozugten Lebensraum durch die überall verstreuten Eier und daraus schlüpfenden Larven laufend neu infizieren.

Die vorliegende Erfindung umfasst somit zwei Aspekte, einmal die bereits beschriebene Methode zur Verhinderung einer Reinfestation von Haustieren mit Flöhen, gleichzeitig natürlich auch die Unterdrückung der Vermehrung von Flöhen.

Erfindungswesentlich ist, dass die Verbindungen der Formel I so verabreicht werden, dass sie vom adulten, saugenden Floh mit dem Blut des Wirtstieres in ausreichender Menge aufgenommen werden können und den adulten Floh rasch töten bevor dieser über seine Ausscheidungen genügend nicht-kontaminierte Nahrung für die Flohlarven bereitstellen kann. Dies wird mit der erfindungsgemässen Wirksubstanzen mit verschiedenen Applikationsformen erreicht, z.B. indem man den formulierten Wirkstoff oral verabreicht. Formuliert heisst in diesem Fall z.B. in Form eines Pulvers, einer Tablette, eines Granulats, einer Kapsel, einer Emulsion, eines Schaumes, im mikroenkapsulierter Form, etc., wobei man das Präparat dem Tier nicht unbedingt direkt zu geben braucht, sondern zweckmässigerweise unter sein Futter mischt, da diese Applikationsform sowohl dem Wirtstier, als auch dem Anwender die wenigsten Unanehmlichkeiten verursacht. Selbstverständlich können alle oral zu verabreichenden Kompositionen neben üblichen Formulierungsstoffen weitere Zusätze enthalten, die die freiwillige Aufnahme durch das Wirtstier fördern, z.B. geeignete Duft- und Aromastoffe. Die orale Verwendung ist, auf Grund ihrer einfachen Ausführbarkeit, eine der bevorzugten Gegenstände dieser Erfindung. Eine weitere Applikationsart ist der parenterale Einsatz, z.B. durch subkutane Injektion oder Injektion in die Vene oder das Langzeitpräparat (Depotform) in Form eines Implantates.

Die orale Applikation schliesst z.B. das Verabreichen von Hunde- und Katzenfutter ein, das die Aktivsubstanz bereits beigemischt enthält, z.B. als Biskuits oder als Leckerbissen. als Kautablette, als wasserlösliche Kapseln oder Tabletten, in wasserlöslicher Form die auf das Futter getropft werden kann oder in sonstigen, dem Tierfutter beimengbaren Formen. Die Implantate schliessen auch alle Vorrichtungen ein, die in den Körper des Tieres zur Substanzabgabe eingebracht werden können.

Durch Wahl einer geeigneten Formulierung ist es möglich das Eindringungsvermögen des Wirkstoffes durch das lebende Gewebe des Tieres zu fördern bzw. seine Verfügbarkeit aufrechtzuerhalten. Dies ist von Bedeutung, wenn z.B. ein sehr schwerlöslicher Wirkstoff eingesetzt wird, dessen geringe Löslichkeit eine löslichkeitsfördernde Massnahme erfordert, da die Körperflüssigkeit des Tieres nur geringe Mengen des Wirkstoffes auf

Die Verabreichung von veterinärmedizinischen Zusätzen zum Tierfutter ist auf dem Gebiet der Tiergesundheit bestens bekannt. Ueblicherweise wird zunächst ein sogenanntes Premix (Vormixtur) hergestellt, in dem die Aktivsubstanz in einer Flüssigkeit dispergiert oder feinverteilt in festen Trägermaterialien vorliegt. Dieses Premix kann normalerweise, in Abhängigkeit von der gewünschten Endkonzentration im Futter, etwa 1 bis 800 g der Substanz pro kg Premix enthalten.

Es ist zudem bekannt, dass Wirkstoffe durch die Bestandteile des Futters hydrolisiert oder geschwächt werden können. Solche Wirksubstanzen werden routinemässig in einer Schutzmatrix, z.B. in Gelatine, formuliert, bevor man sie dem Premix zusetzt.

Folglich betrifft die vorliegende Erfindung den Aspekt der Beseitigung der adulten Flöhe auf dem Haustier, als auch die Hinderung der Flohlarven an einer Weiterentwicklung durch Nahrungsmittelentzug, was einer systemischen Vorbeugung vor einer Reinfektion von Haustieren, insbesondere von Haustieren durch Flöhe gleichkommt. Dies wird dadurch erreicht, dass man dem besagten Wirtstier oral, eine gegen Flöhe wirksame Menge mindestens einer Verbindung der Formel I zusetzt.

Die vorliegende Erfindung betrifft somit auch den Aspekt der Verhütung der Vermehrung von Flöhen, der sich dadurch kennzeichnet, dass man Flöhen, über die systemische Verabreichung der Wirksubstanz an das Wirtstier, als Nahrungsmittel kontaminiertes Blut zur Verfügung stellt, das eine gegen Flöhe wirksame Menge mindestens einer Verbindung der Formel I enthält. Dies wird am einfachsten erreicht indem man eine gegen Flöhe wirksame Menge Verbindung der Formel I dem Wirtstier in Form von Futterzusatz verabreicht und auf diese Weise an die auf dem Wirtstier lebenden Flöhe gelangen lässt.

Die Verbindungen der Formel I werden zweckmässigerweise in einer Dosierung von 0,01 bis 800, vorzugsweise 0,1 bis 200, insbesondere 0,5 bis 30 mg/kg Körpergewicht in Bezug auf das Wirtstier appliziert, wobei die orale Verabreichung bevorzugt ist.

Eine gute Dosis, die regelmässig an das Wirtstier verabreicht werden kann, liegt bei 0,5 bis 100 mg/kg Körpergewicht. Die Verabreichung erfolgt zweckmässigerweise täglich oder wöchentlich.

Die Gesamtdosis kann bei gleichem Wirkstoff von einer Tiergattung zur anderen und auch innerhalb einer Tiergattung variieren, da sie u.a. vom Gewicht und der Konstitution des Tieres abhängt.

Bei der erfindungsgemässen Verwendung wird der Wirkstoff normalerweise nicht in reiner Form appliziert, sondern vorzugsweise in Form eines Mittels, das neben dem Wirkstoff applikationsfördernde Bestandteile enthält, wobei solche Bestandteile in Frage kommen, die dem Wirtstier zuträglich sind. Selbstverständlich können neben der erfindungsgemässen Bekämpfung der adulten Flöhe zusätzlich mit konventionellen Methoden, die die juvenilen Flohstadien bekämpft werden, letzteres ist jedoch nicht zwingend notwendig.

Derartige erfindungsgemäss anzuwendende Mittel enthalten in der Regel 0,1 bis 99 Gew.-%, insbesondere 0,1 bis 95 Gew.-%, einer Verbindung der Formel I und 99,9 bis 1 Gew.-%, insbesondere 99,9 bis 5 Gew.-%, eines festen oder flüssigen, untoxischen Zusatzstoffes, darunter 0 bis 25 Gew.-%, insbesondere 0,1 bis 25 Gew.-%, eines untoxischen Tensides.

Während als Handelsware eher konzentrierte Mittel bevorzugt werden, verwendet der Endverbraucher in der Regel verdünnte Mittel.

Solche Mittel können noch weitere Zusätze wie Stabilisatoren, Entschäumer, Viskositätsregulatoren, Bindemittel, Haftmittel sowie andere Wirkstoffe zur Erzielung spezieller Effekte enthalten.

Als Formulierungshilfsstoffe können die aus der veterinärmedizinischen Praxis für orale Mittel bekannten Materialien eingesetzt werden. Nachfolgend seien einige Beispiele genannt.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinyl-pyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne können mit geeigneten, gegebenenfalls magensaftresistenten, Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, oder Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe, Aromastoffe oder Pigmente, z.B. zur Identifizierung oder zur Kennzeichnung verschiedener Wirkstoffdosen, beigefügt werden.

Weitere oral anwendbare Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Starken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können. Bevorzugt sind u.a. Kapseln, die sowohl leicht zerbissen als auch unzerkaut geschluckt werden können.

Die Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren Umfang in keiner Weise ein. Temperaturen werden in Celsiusgraden angegeben.

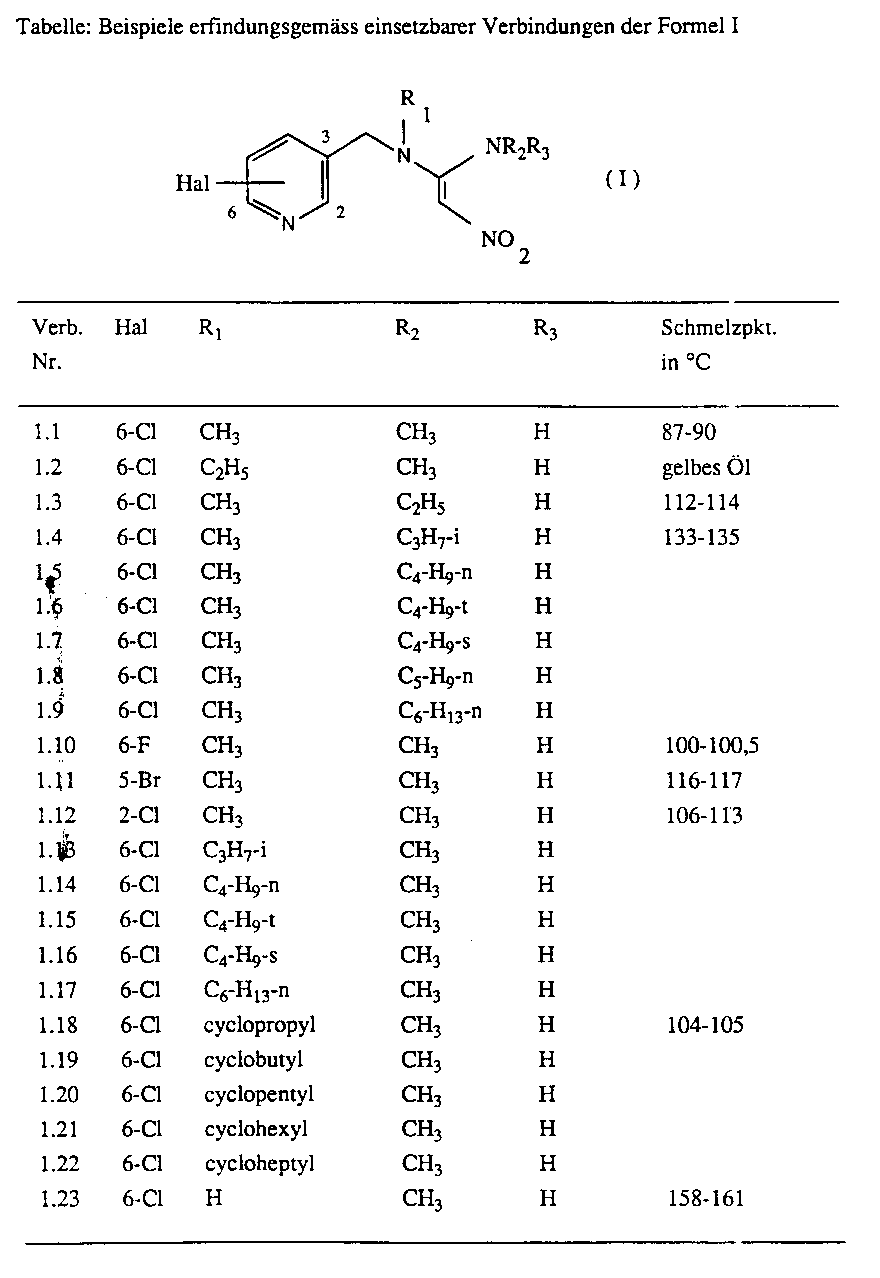

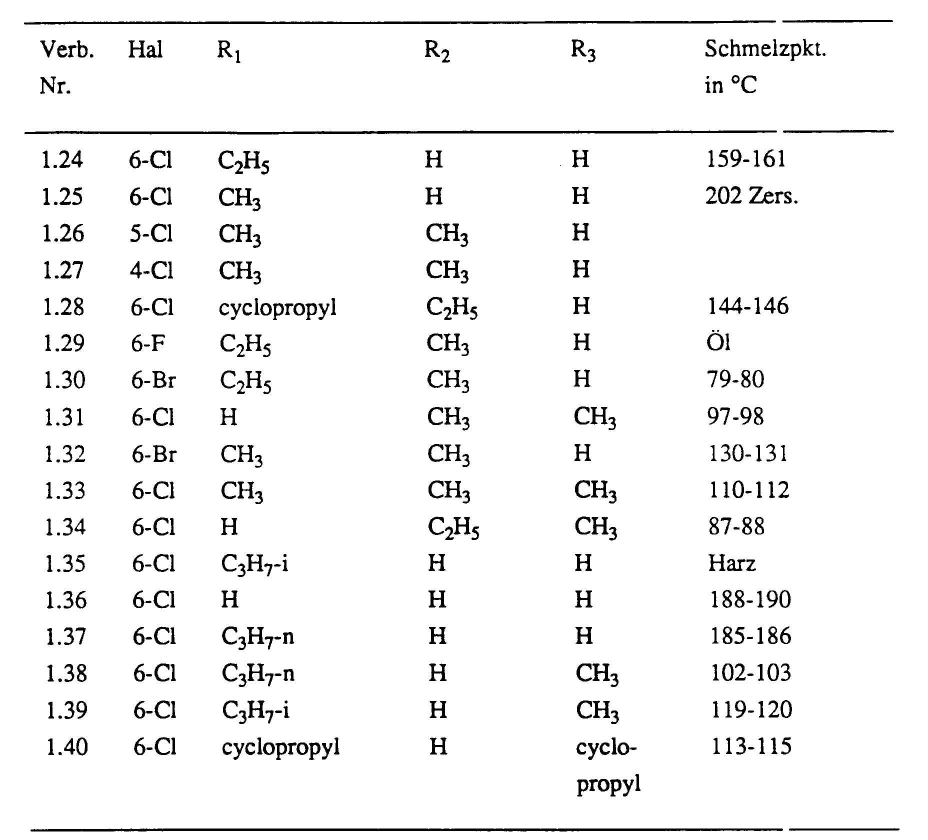

In den nachfolgenden Formulierungsbeispielen steht der Begriff Wirkstoff stellvertretend für 1-[N-(6-Chlor-3-pyridylmethyl)]-N-ethylamino-1-methylamino-2-nitroethylen.

### Beispiel 1: Tabletten enthaltend 25 mg des Wirkstoffs können folgendermassen hergestellt werden:

### Bestandteile (für 1000 Tabletten)

- Wirkstoff: 25,0 g
- Lactose: 100,7 g
- Weizenstärke: 7,5 g
- Polyethylenglykol 6000: 5,0 g
- Talkum: 5,0 g
- Magnesiumstearat: 1,8 g
- entmineralisiertes Wasser: q.s.

Herstellung: Sämtliche festen Ingredienzien werden zunächst durch ein Sieb mit 0,6 mm Maschenweite getrieben. Dann wird der Wirkstoff, die Lactose, das Talkum und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben, mit dem Magnesiumstearat vermischz und zu beidseitig konkaven Tabletten von etwa 6 mm Maschenweite verpresst.

### Beispiel 2: Tabletten enthaltend 0,02 g Wirkstoff werden wie folgt hergestellt:

### Zusammensetzung (für 10'000 Tabletten)

- Wirkstoff: 200,00 g
- Lactose: 290,80 g
- Kartoffelstärke: 274,70 g
- Stearinsäure: 10,00 g
- Talk: 200,00 g
- Magnesiumstearat: 2,50 g
- Kolloidales Siliciumdioxid: 32,00.
- Ethanol: q.s.

Ein Gemisch des Wirkstoffs, der Lactose und 194,70 g Kartoffelstärke wird mit einer ethanolischen Lösung der Stearinsäure befeuchtet und durch ein Sieb granuliert. Nach dem Trocknen mischt man die restliche Kartoffelstärke, den Talk, das Magnesiumstearat und das kolloidale Siliciumdioxid zu und presst die Mischung zu Tabletten von je 0,1 g Gewicht, die gewünschtenfalls mit Teilkerben zur feineren Anpassung der Dosierung versehen werden können.

### Beispiel 3: Kapseln, enthaltend 0,025 g des Wirkstoffs können wie folgt hergestellt werden:

### Zusammensetzung (für 1000 Kapseln)

- Wirkstoff: 25,00 g
- Lactose: 249,80 g
- Gelatine: 2,00 g
- Maisstärke: 10,00 g
- Talk: 15,00 g
- Wasser: q.s.

Man mischt den Wirkstoff mit der Lactose, befeuchtet die Mischung gleichmässig mit einer wässrigen Lösung der Gelatine und granuliert sie durch ein Sieb mit einer Maschenweite von 1,2-1,5 mm. Das Granulat mischt man mit der getrockneten Maisstärke und dem Talk und füllt Portionen von 300 mg in Hartgelatinekapseln (Grösse 1) ab.

### Beispeil 4: Premix (Futterzusatzmittel)

- 0,25: Gewichtsteile Wirkstoff und
- 4,75: Gewichtsteile sekundäres Calciumphosphat, Tonerde, Aerosil, Carbonat oder Kalk werden bis zur Homogenität mit
- 95: Gewichtsteilen eines Tierfutters gemischt.

### Beispiel 5: Premix (Futterzusatzmittel)

- 0,40: Gewichtsteile Wirkstoff und
- 5,00: Gewichtsteile Aerosil/Kalk (1:1) werden bis zur Homogenität mit
- 94,6: Gewichtsteilen eines käuflichen Trockenfutters gemischt.

### Beispiel 6: Emulgierkonzentrat

- 20: Gewichtsteile des Wirkstoffs werden mit
- 20: Gewichtsteilen des Emulgators, z.B. eines Gemisches von Alkylarylpolyglykolether mit Alkylarylpolysulfonaten, und mit
- 60: Gewichtsteilen eines Lösungsmittels so lange gemischt, bis die Lösung vollständig homogenisiert ist. Durch Verdünnen mit Wasser erhält man Emulsionen gewünschter Konzentration.

### Beispiel 7: Lösungen (z.B. zur Verwendung als Trinkzusatz)

- 15: Gewichtsprozent des Wirkstoffs in 2,2-Dimethyl-4-hydroxymethyl-1,3-dioxolan,
- 10: Gewichtsprozent des Wirkstoffs in Diethylenglykolmonoethylether,
- 10: Gewichtsprozent in Polyethylenglykol 300, und
- 5: Gewichtsprozent in Glyzerol.

### Beispiel 8: Lösliches Pulver

- 25: Gewichtsteile Wirkstoff
- 1: Gewichtsteil Natriumlaurylsulfat,
- 3: Gewichtsteile kolloides Kieselgel, und
- 71: Gewichtsteile Harnstoff.

Die Bestandteile werden gemischt und bis zur Homogenität miteinander vermahlen.

Es können weitere biologisch aktive Substanzen oder Zusätze, die sich gegenüber den Wirkstoffen neutral verhalten und keinen schädlichen Einfluss auf das zu behandelnde Wirtstier haben, sowie Mineralsalze oder Vitamine den beschriebenen Kompositionen zugesetztwerden.

### Biologische Beispiele

### Beispiel 9: Vergleichsversuch zur Wirkung gegen Ctenocephalides felis (Katzenfloh)

Gemäss dem nachfolgenden Protokoll wurden sowohl die erfindungsgemässe Wirksubstanz 1-[N-(6-Chlor-3-pyridylmethyl)]-N-ethylamino-1-methylamino-2-nitroethylen mit der chemischen Struktur: 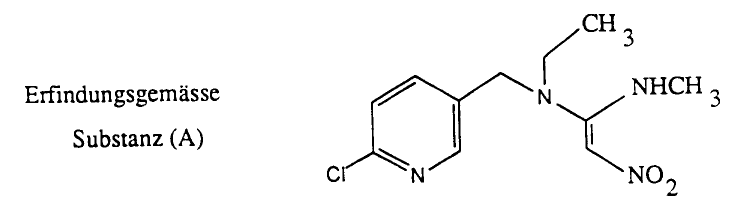 als auch der strukturell nächstvergleichbare in EP-0'302'833 genannte Vertreter der Formel: 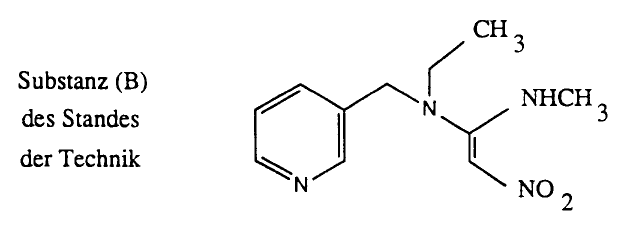 vergleichend gegen eine unbehandelte Kontrollgruppe von Flöhen getestet.

### Versuchsprotokoll:

20 adulte Flöhe der Art Ctenocephalides felis werden in einen flachen runden Käfig gegeben, der auf beiden Seiten mit Gaze verschlossen ist. Auf diesen Käfig wird nun ein Gefäss gestellt, das auf der unteren Seite mit einer Parafilmmembran verschlossen ist. Das Gefäss enthält Blut, das 1,0 ppm des Wirkstoffes enthält und konstant auf 37°C erwärmt wird. Die Flöhe nehmen das Blut durch die Membran auf. 24 und 48 Stunden nach Ansatz erfolgt die Auswertung. Aus dem Vergleich der Anzahl toter Flöhe mit behandeltem Blut zu denjenigen mit unbehandeltem Blut (Kontrollgruppe) wird die prozentuale Reduktion der Population (% Wirkung) bestimmt. 24 Stunden nach Behandlung wird das Blut durch neues ebenfalls behandeltes Blut ersetzt und der Versuch mit den überlebenden Flöhen fortgesetzt. Auch das unbehandelte Blut der Kontrollgruppe wird nach 24 Stunden ersetzt.

Resultate: Alle drei Gruppen von Flöhen [(Kontrollgruppe / unbehandeltes Blut); (Gruppe A / Blut behandelt mit der erfindungsgemässen Substanz A) und (Gruppe B / Blut behandelt mit der Substanz B aus dem Stand der Technik)] begannen sofort mit der Blutaufnahme sobald sie mit in die Testvorrichtung gesetzt wurden. Das Verhalten der Kontrollgruppe und der Gruppe B (St.d.T.) bleiben über den Versuchszeitraum praktisch unverändert. Demgegenüber zeigten sich bereits eine halbe Stunde nach Versuchsbeginn bei der Gruppe A (erfindungsgemässe Behandlung) die ersten toxischen Erscheinungen. Nach 24 Stunden wurden folgende Einzelresultate beim Einsatz von je 2x20 Flöhen festgestellt (in % Mortalität):

| Substanz | ppm A.S. | Test 1 | Test 2 | Test 3 | Test 4 |
|---|---|---|---|---|---|
| B | 1.0 | 0 % | 5 % | 0 % | 0 % |
| A | 1.0 | 100 & | 95 % | 95 % | 100 % |
| Kontroll | 0,0 | 0 % | 0 % | 0 % | 0 % |

### Beispiel 10: In vivo-Vergleichsversuch zur Wirkung gegen Ctenocephalides felis (Katzenfloh)

In dieser vergleichenden Untersuchung wurden wie in Beispiel 9 wiederum die erfindungsgemässe Substanz A mit der strukturell nächstvergleichbaren Substanz B des Standes der Technik vergleichend an Katzen geprüft.

Prüft man andere Substanzen aus der Tabelle mit den beispielhaft angegebenen Verbindungen der Formel I, so kommt man zu absolut vergleichbaren Resultaten.

### Versuchsprotokoll:

6 weibliche Hauskatzen im Alter von 1-2 Jahren mit einem Körpergewicht zwischen 3,0 und 4,0 kg wurden in drei Gruppen mit je zwei Tieren eingeteilt. Eine Gruppe wurde zwar ebenfalls mit Flöhen ifiziert, blieb aber unbehandelt und diente als Kontrollgruppe. Eine der übrigen Gruppen erhielt die Prüfsubstanz A, die andere die Substanz B jeweils in einer Dosierung von 10 mg/Kg Körpergewicht mittels einer Gelatinekapsel direkt in den Schlund. Jede der Aktivsubstanzen wurde vorher mit Laktose 1:1 vermischt. Unmittelbar nach Verabreichung der Wirksubstanzen wurden die Katzen in der Gegend des Widerristes mit je 20 Flöhen (16 weibliche und 4 männliche Flöhe) infestiert. Weitere Flohinfestationen mit weiteren 20 Flöhen erfolgten bei der Substanz A am Tage +2, +4 und +6. Bei den Katzen, die mit Substanz B behandelt wurden und bei der Vergleichsgruppe, war eine weitere Infestation mit Flöhen nicht erforderlich, da alle Flöhe von der Erstinfestation überlebt hatten.
Die Floheier wurden täglich eingesammelt und gezählt. Die Zahl der auffindbaren getöteten Flöhe wurde ebenfalls täglich bestimmt. Die Resultate der 2 Behandlungsgruppen wurden sowohl untereinander, als auch mit dem der Kontrollgruppe verglichen.

Resultate: Die Zahl der auffindbaren getöteten Flöhe sowie die Eiproduktion sind in den nachfolgenden Tabellen 1 und 2 wiedergegeben:

**Tabelle 1**

| Zahl der getöteten Flöhe | | | | | | | | | | | |
|---|---|---|---|---|---|---|---|---|---|---|---|
| Substanz | Katze-Nr. | Getötete Flöhe am Tage... | | | | | | | | | |
| | | 0 | +1 | +2 | +3 | +4 | +5 | +6 | +7 | +8 | +9 |
| A | 325 | 12* | 1 | 0* | 11 | 5* | 0 | 2* | 0 | 0 | 0 |
| | 343 | 9* | 1 | 0* | 8 | 2* | 0 | 2* | 0 | 0 | 0 |
| B | 351 | 0* | 1 | 0 | 0 | 0 | 0 | 0 | 0 | 0 | 0 |
| | 266 | 0* | 0 | 0 | 0 | 0 | 0 | 0 | 0 | 0 | 0 |
| Kontrolle | 339 | 0* | 0 | 0 | 0 | 0 | 0 | 0 | 0 | 0 | 0 |
| | 599 | 0* | 0 | 0 | 0 | 0 | 0 | 0 | 0 | 0 | 0 |

| | | | | | | | | | | | |
|---|---|---|---|---|---|---|---|---|---|---|---|
| * Infestation mit 20 Flöhen | | | | | | | | | | | |

Bei der Substanz A wurde wurden bereits 6 Stunden nach Infestation mit den ersten 20 Flöhen 60% abgetötet. Selbst nach am zweiten Tag nach der erneuten Infestation mit weiteren 20 Flöhen wurden noch 55% der Flöge abgetötet. Die Wirkung der Substanz A sank erst am Tage 6 auf ca. 10% ab. Demgegenüber war zwischen der Kontrollgruppe und der Gruppe von Katzen, die mit Substanz B behandelt worden waren kein signifikanter Unterschied erkennbar. Die Substanz B erwies sich bei den geringen Testkonzentrationen als unwirksam und für eine orale Verabreichung gegen Flöhe als unbrauchbar.

**Tabelle 2**

| Eiproduktion | | | | | | | | | |
|---|---|---|---|---|---|---|---|---|---|
| Substanz | Katze-Nr. | Eiproduktion am Tage... | | | | | | | |
| | | +2 | +3 | +4 | +5 | +6 | +7 | +8 | +9 |
| A | 325 | 0* | 0 | 0* | 0 | 37* | 233 | 250 | 370 |
| | 343 | 0* | 0 | 0* | 60 | 0* | 186 | 240 | 283 |
| B | 351 | 240 | 306 | 403 | 423 | 350 | 333 | 343 | 290 |
| | 266 | 106 | 266 | 386 | 313 | 286 | 290 | 326 | 303 |
| Kontrolle | 339 | 60 | 133 | 186 | 300 | 310 | 313 | 290 | 233 |
| | 599 | 96 | 110 | 156 | 146 | 166 | 116 | 200 | 170 |

| | | | | | | | | | |
|---|---|---|---|---|---|---|---|---|---|
| * Infestation mit 20 Flöhen | | | | | | | | | |

Bei der mit Substanz A behandelten Gruppe waren nach der ersten und zweiten Flohinfestation keine Floheier auffindbar. Bei der mit Substanz B behandelten Gruppe von Katzen und bei der Kontrollgruppe produzierten die weiblichen Flöhe die übliche Zahl von Eiern; es waren keine signifikanten Unterschiede erkennbar.

Der Vergleichsversuch zeigt, dass Substanz A eine gute systemische Wirkung gegen adulte Flöhe für die Dauer von ca. 3-4 Tagen bewirkt. Substanz B kann hingegen bei der Testdosis als völlig unwirksam eingestuft werden. Es konnte weder vorhergesagt noch erwartet werden, dass die einfache Halogenierung der Pyridylgruppe eine derart signifikante Wirkungssteigerung hinsichtlich der systemischen Antiflohwirkung bringen würde.

Die Verbindungen, die in der Tabelle der erfindungsgemäss einsetzbaren Substanzen mit physikalischen Daten angegeben sind, zeigen eine vergleichbare Antiflohwirkung wie die erfindungsgemässe Substanz A. Auffällig ist vor allem die hervorragende Wirkung der Verbindungen 1.1 bis 1.4, 1.10 bis 1.12, 1.18, 1.28, 1.29, und 1.36 bis 1.40.

## Patentansprüche

1. Verwendung einer Verbindung der Formel 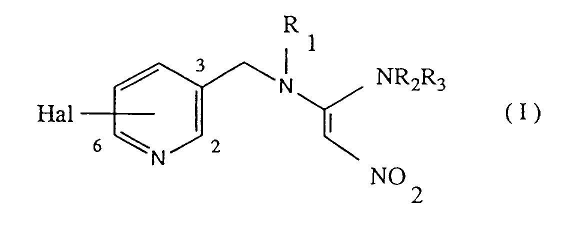 worin
Hal für Halogen, wie Fluor, Chlor, Brom oder Jod steht;
R₁ Wasserstoff, C₁-C₆-Alkyl oder C₃-C₇-Cycloalkyl bedeutet;
R₂ für Wasserstoff, C₁-C₆-Alkyl oder C₃-C₇-Cycloalkyl steht und
R₃ für Wasserstoff oder C₁-C₆-Alkyl steht, zur Herstellung eines Mittels zur systemischen Bekämpfung von adulten Flöhen auf Haustieren.

2. Verwendung einer Verbindung nach Anspruch 1 der Formel I, dadurch gekennzeichnet, dass es sich bei dem Wirkstoff um eine Verbindung der Formel I handelt, worin Hal in der 6-Position steht und vorzugsweise für Fluor, Chlor oder Brom steht, insbesondere jedoch Chlor bedeutet.

3. Verwendung einer Verbindung nach Anspruch 1 der Formel I, dadurch gekennzeichnet, dass es sich bei dem Wirkstoff um eine Verbindung der Formel I handelt, worin R₁ für Wasserstoff, C₁-C₃-Alkyl oder C₃-C₆-Cycloalkyl , vorzugsweise für Wasserstoff, Methyl oder Cyclopropyl, insbesondere für Methyl steht.

4. Verwendung einer Verbindung nach Anspruch 1 der Formel I, dadurch gekennzeichnet, dass es sich bei dem Wirkstoff um eine Verbindung der Formel I handelt, worin R₂ für C₁-C₃-Alkyl oder Cyclopropyl, insbesondere für Methyl steht.

5. Verwendung einer Verbindung nach Anspruch 1 der Formel I, dadurch gekennzeichnet, dass es sich bei dem Wirkstoff um eine Verbindung der Formel I nach einem der Ansprüche 2 bis 4 handelt, worin R₃ für Wasserstoff steht.

6. Verwendung einer Verbindung nach Anspruch 1 der Formel I, dadurch gekennzeichnet, dass es sich bei dem Haustier um einen Hund oder eine Katze handelt.

7. Verwendung einer Verbindung nach Anspruch 6 der Formel I, dadurch gekennzeichnet, dass man als begleitende Massnahme die juvenilen Flohstadien mit herkömmlichen larvizid oder ovizid wirkenden Antiflohmittel bekämpft.

8. Verwendung einer Verbindung nach einem der Ansprüche 1 bis 7 der Formel I, dadurch gekennzeichnet, dass man ein den Wirkstoff enthaltendes Mittel oral in regelmässigen Einzeldosen verabreicht.

9. Verwendung einer Verbindung nach einem der Ansprüche 1 bis 7 der Formel I, dadurch gekennzeichnet, dass man die Aktivsubstanz dem Wirtstier in Aufwandmengen von etwa 0,01 mg/kg Körpergewicht bis etwa 800 mg/kg Körpergewicht verabreicht.

10. Verwendung einer Verbindung nach Anspruch 9 der Formel I, dadurch gekennzeichnet, dass man die Aktivsubstanz dem Wirtstier in Aufwandmengen von etwa 0,1 mg/kg Körpergewicht bis etwa 200 mg/kg Körpergewicht verabreicht.

11. Verwendung einer Verbindung nach Anspruch 10, dadurch gekennzeichnet, dass man die Aktivsubstanz dem Wirtstier in Aufwandmengen von etwa 0,5 mg/kg Körpergewicht bis etwa 30 mg/kg Körpergewicht verabreicht.

12. Verwendung einer Verbindung nach einem der Ansprüche 9 bis 10 der Formel I, dadurch gekennzeichnet, dass man den Wirkstoff der Katze oder dem Hund oral verabreicht.

13. Verwendung einer Verbindung nach einem der Ansprüche 1 bis 7 der Formel I, dadurch gekennzeichnet, dass man der Katze oder dem Hund regelmässig eine Dosis von 0,5 mg/kg Körpergewicht bis etwa 100 mg/kg Körpergewicht der Aktivsubstanz verabreicht.

14. Verwendung einer Verbindung der Formel 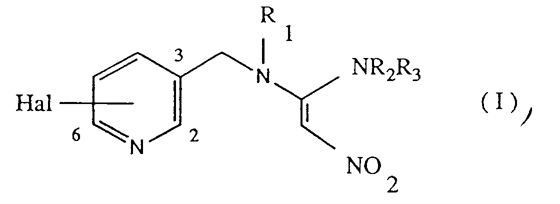 worin
Hal für Halogen, wie Fluor, Chlor, Brom oder Jod steht;
R₁ Wasserstoff, C₁-C₆-Alkyl oder C₃-C₇-Cycloalkyl bedeutet;
R₂ für Wasserstoff, C₁-C₆-Alkyl oder C₃-C₇-Cycloalkyl steht und
R₃ für Wasserstoff oder C₁-C₆-Alkyl steht , zur Herstellung eines Mittels zur systemischen Verabreichung an das Blut von Haustieren zur Verhütung der Vermehrung von Flöhen auf dem besagten Haustier.

15. Verwendung einer Verbindung nach Anspruch 14 der Formel I, dadurch gekennzeichnet, dass es sich bei dem Wirkstoff um eine der in den Ansprüchen 2 bis 4 definierten Verbindungen der Formel I handelt.

16. Verwendung einer Verbindung nach Anspruch 14 der Formel I, dadurch gekennzeichnet, dass man eine wirksame Menge der Aktivsubstanz mit dem Futter dem Wirtstier verabreicht und diese durch die auf dem Wirtstier befindlichen Flöhe mit dem gesaugten Blut aufnehmen lässt.

17. Systemisch wirkendes Mittel zur Verhütung eines Befalls von Haustieren, vorzugsweise von Hunden und Katzen durch Flöhe, dadurch gekennzeichnet, dass es eine gegen Flöhe wirksame Menge einer Verbindung der Formel I 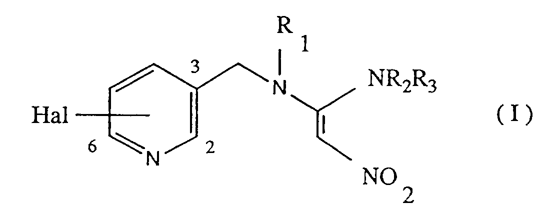 worin
Hal für Halogen, wie Fluor, Chlor, Brom oder Jod steht;
R₁ Wasserstoff, C₁-C₆-Alkyl oder C₃-C₇-Cycloalkyl bedeutet;
R₂ für Wasserstoff, C₁-C₆-Alkyl oder C₃-C₇-Cycloalkyl steht und
R₃ für Wasserstoff oder C₁-C₆-Alkyl steht, zusammen mit Tierphysiologisch verträglichen Hilfsstoffen im Futter enthält.

18. Mittel nach Anspruch 17, dadurch gekennzeichnet, dass es sich um Hunde- oder Katzenfutter handelt.

19. Mittel nach Anspruch 17, dadurch gekennzeichnet, dass es sich bei dem Wirkstoff um eine Verbindung der Formel I gemäss einem der Ansprüche 2 bis 4 handelt.

20. Futterzusatzmittel für die systemische Bekämpfung von Flöhen bei Haustieren, dadurch gekennzeichnet, dass es als Aktivsubstanz eine Verbindung der Formel I 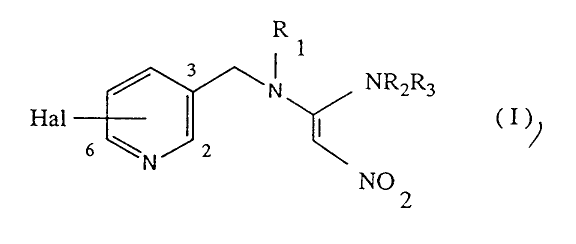 worin
Hal für Halogen, wie Fluor, Chlor, Brom oder Jod steht;
R₁ Wasserstoff, C₁-C₆-Alkyl oder C₃-C₇-Cycloalkyl bedeutet;
R₂ für Wasserstoff, C₁-C₆-Alkyl oder C₃-C₇-Cycloalkyl steht und
R₃ für Wasserstoff oder C₁-C₆-Alkyl steht, in einer gegen Flöhe aktiven Menge enthält.

21. Packung enthaltend Einzeldosen von einer Verbindung der Formel I 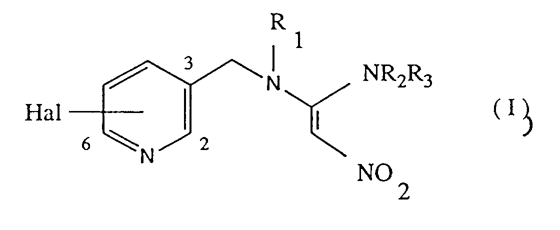 worin
Hal für Halogen, wie Fluor, Chlor, Brom oder Jod steht;
R₁ Wasserstoff, C₁-C₆-Alkyl oder C₃-C₇-Cycloalkyl bedeutet;
R₂ für Wasserstoff, C₁-C₆-Alkyl oder C₃-C₇-Cycloalkyl steht und
R₃ für Wasserstoff oder C₁-C₆-Alkyl steht in formulierter Form für die orale Anwendung gegen Flöhe beim Haustier, dadurch gekennzeichnet, dass der Wirkstoff in gebrauchsfertigen Einzeldosen von 0,01 mg/kg Körpergewicht bis etwa 800 mg/kg Körpergewicht vorliegt.

## Claims

1. Use of a compound of formula 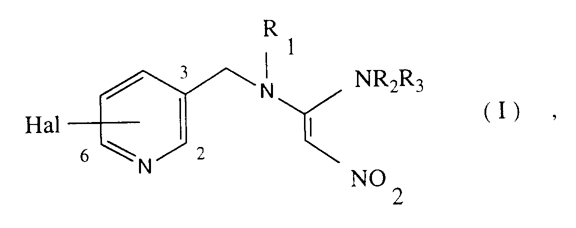 wherein
Hal is halogen, such as fluorine, chlorine, bromine or iodine;
R₁ is hydrogen, C₁-C₆alkyl or C₃-C₇cycloalkyl;
R₂ is hydrogen, C₁-C₆alkyl or C₃-C₇cycloalkyl, and
R₃ is hydrogen or C₁-C₆alkyl,
in the preparation of a composition for the systemic control of adult fleas on domestic animals.

2. Use of a compound according to claim 1 of formula I wherein the active ingredient is a compound of formula I wherein Hal is in the 6-position and is preferably fluorine, chlorine or bromine, but especially chlorine.

3. Use of a compound according to claim 1 of formula 1 wherein the active ingredient is a compound of formula I wherein R₁ is hydrogen, C₁-C₃alkyl or C₃-C₆cycloalkyl preferably hydrogen, methyl or cyclopropyl, especially methyl.

4. Use of a compound according to claim 1 of formula I wherein the active ingredient is a compound of formula I wherein R₂ is C₁-C₃alkyl or cyclopropyl, especially methyl.

5. Use of a compound according to claim 1 of formula I wherein the active ingredient is a compound of formula I according to any one of claims 2 to 4 wherein R₃ is hydrogen.

6. Use of a compound according to claim 1 of formula I, wherein the domestic animal is a dog or a cat.

7. Use of a compound according to claim 6 of formula I, which comprises, as an accompanying measure, controlling the juvenile flea stages using conventional larvicidal or ovicidal anti-flea compositions.

8. Use of a compound according to any one of claims 1 to 7 of formula I, which comprises administering orally in regular individual doses a composition comprising the active ingredient.

9. Use of a compound according to any one of claims 1 to 7 of formula I, which comprises administering the active ingredient to the host animal in concentrations of from approximately 0.01 mg/kg of body weight to approximately 800 mg/kg of body weight.

10. Use of a compound according to claim 9 of formula I, which comprises administering the active ingredient to the host animal in concentrations of from approximately 0.1 mg/kg of body weight to approximately 200 mg/kg of body weight.

11. Use of a compound according to claim 10, which comprises administering the active ingredient to the host animal in concentrations of from approximately 0.5 mg/kg of body weight to approximately 30 mg/kg of body weight.

12. Use of a compound according to either claim 9 or claim 10 of formula I, which comprises administering the active ingredient to the cat or the dog orally.

13. Use of a compound according to any one of claims 1 to 7 of formula I, which comprises administering the active ingredient regularly to the cat or the dog in a dose of from 0.5 mg/kg of body weight to approximately 100 mg/kg of body weight.

14. Use of a compound of formula 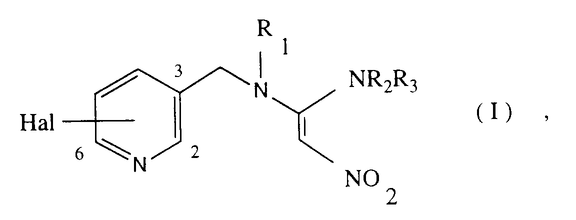 wherein
Hal is halogen, such as fluorine, chlorine, bromine or iodine;
R₁ is hydrogen, C₁-C₆alkyl or C₃-C₇cycloalkyl;
R₂ is hydrogen, C₁-C₆alkyl or C₃-C₇cycloalkyl and
R₃ is hydrogen or C₁-C₆alkyl
in the preparation of a composition for systemic administration to the blood of domestic animals for preventing fleas from reproducing on the said domestic animal.

15. Use of a compound according to claim 14 of formula I, wherein the active ingredient is one of the compounds of formula I defined in claims 2 to 4.

16. Use of a compound according to claim 14 of formula I, which comprises administering an effective amount of the active ingredient to the host animal with its food and allowing the active ingredient to be taken up by the fleas on the host animal with the blood they suck up.

17. A systemically active composition for preventing the infestation of domestic animals, especially of dogs and cats by fleas, which comprises in food, in an amount effective against fleas, a compound of formula I 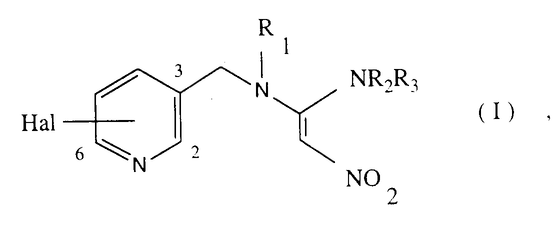 wherein
Hal is halogen, such as fluorine, chlorine, bromine or iodine;
R₁ is hydrogen, C₁-C₆alkyl or C₃-C₇cycloalkyl;
R₂ is hydrogen, C₁-C₆alkyl or C₃-C₇cycloalkyl and
R₃ is hydrogen or C₁-C₆alkyl,
together with excipients that are physiologically tolerable for animals.

18. A composition according to claim 17 comprising dog or cat food.

19. A composition according to claim 17, wherein the active ingredient is a compound of formula I according to any one of claims 2 to 4.

20. Food additive for the systemic control of fleas in domestic animals, which comprises as active ingredient, in an amount effective against fleas, a compound of formula I 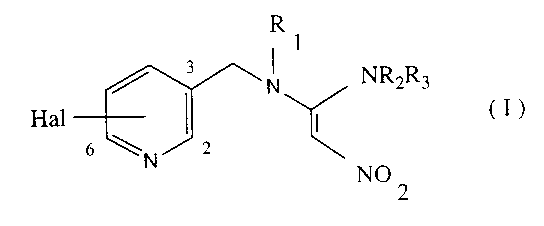 wherein
Hal is halogen, such as fluorine, chlorine, bromine or iodine;
R₁ is hydrogen, C₁-C₆alkyl or C₃-C₇cycloalkyl;
R₂ is hydrogen, C₁-C₆alkyl or C₃-C₇cycloalkyl, and
R₃ is hydrogen or C₁-C₆alkyl.

21. A pack containing individual doses of a compound of formula I 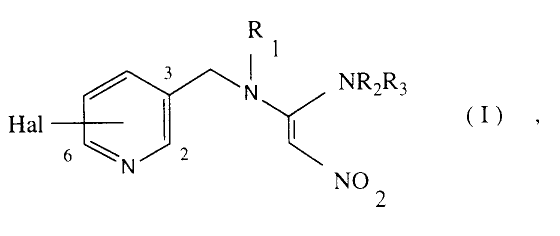 wherein
Hal is halogen, such as fluorine, chlorine, bromine or iodine;
R₁ is hydrogen, C₁-C₆alkyl or C₃-C₇cycloalkyl;
R₂ is hydrogen, C₁-C₆alkyl or C₃-C₇cycloalkyl and
R₃ is hydrogen or C₁-C₆alkyl,
in formulated form for oral administration against fleas in domestic animals, wherein the active ingredient is present in individual ready-for-use doses of from 0.01 mg/kg of body weight to approximately 800 mg/kg of body weight.

## Revendications

1. Utilisation d'un composé de formule 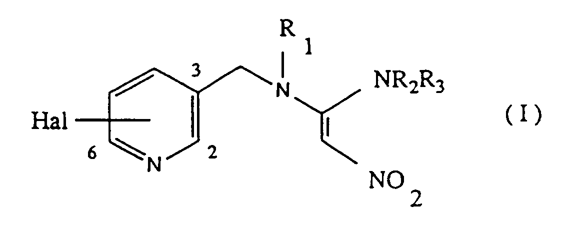 où
Hal signifie un halogène tel que le fluor, le chlore, le brome ou l'iode;
R₁ signifie l'hydrogène, un groupe C₁-C₆-alkyle ou C₃-C₇cycloalkyle;
R₂ signifie l'hydrogène, un groupe C₁-C₆-alkyle ou C₃-C₇cycloalkyle et
R₃ signifie l'hydrogène ou un groupe C₁-C₆-alkyle , pour la préparation d'un agent
pour la lutte systémique contre les puces adultes sur des animaux domestiques.

2. Utilisation d'un composé selon la revendication 1 de formule I, caractérisée en ce que la matière active est un composé de formule I, où Hal est situé en position 6 et signifie de préférence le fluor, le chlore ou le brome, en particulier le chlore.

3. Utilisation d'un composé selon la revendication 1 de formule I, caractérisée en ce que la matière active est un composé de formule I, où R₁ signifie l'hydrogène, un groupe C₁-C₃-alkyle ou C₃-C₆-cycloalkyle, de préférence l'hydrogène, un groupe méthyle ou cyclopropyle, en particulier un groupe méthyle.

4. Utilisation d'un composé selon la revendication 1 de formule I, caractérisée en ce que la matière active est un composé de formule I, où R₂ signifie un groupe C₁-C₃-alkyle ou cyclopropyle, en particulier un groupe méthyle.

5. Utilisation d'un composé selon la revendication 1 de formule I, caractérisée en ce que la matière active est un composé de formule I selon l'une des revendications 2 à 4, où R₃ signifie l'hydrogène.

6. Utilisation d'un composé selon la revendication 1 de formule I, caractérisée en ce que l'animal domestique est un chien ou un chat.

7. Utilisation d'un composé selon la revendication 6 de formule I, caractérisée en ce que, comme mesure d'accompagnement, on lutte contre les stades juvéniles des puces avec un agent anti-puce habituel agissant comme larvicide ou ovicide.

8. Utilisation d'un composé selon l'une des revendications 1 à 7 de formule I, caractérisée en ce que l'agent contenant la matière active est administré par voie orale dans des doses unitaires régulières.

9. Utilisation d'un composé selon l'une des revendications 1 à 7 de formule I, caractérisée en ce qu'on administre la matière active à l'animal hôte en quantités comprises entre environ 0,01 mg/kg de poids corporel et environ 800 mg/kg de poids corporel.

10. Utilisation d'un composé selon la revendication 9 de formule I, caractérisée en ce qu'on administre la matière active à l'animal hôte en quantités comprises entre environ 0,1 mg/kg de poids corporel et environ 200 mg/kg de poids corporel.

11. Utilisation d'un composé selon la revendication 10, caractérisée en ce qu'on administre la matière active à l'animal hôte en quantités comprises entre environ 0,5 mg/kg de poids corporel et environ 30 mg/kg de poids corporel.

12. Utilisation d'un composé selon l'une des revendications 9 à 10 de formule I, caractérisée en ce qu'on administre la matière active au chat ou au chien par voie orale.

13. Utilisation d'un composé selon l'une des revendications 1 à 7 de formule I, caractérisée en ce qu'on administre régulièrement au chat ou au chien une dose de matière active comprise entre 0,5 mg/kg de poids corporel et environ 100 mg/kg de poids corporel.

14. Utilisation d'un composé de formule 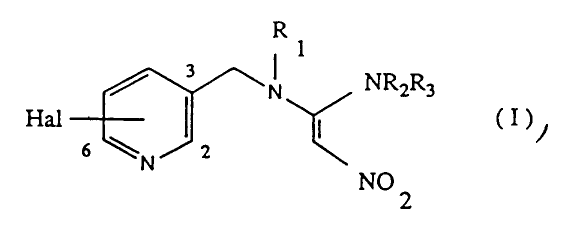 où
Hal signifie un halogène tel que le fluor, le chlore, le brome ou l'iode;
R₁ signifie l'hydrogène, un groupe C₁-C₆-alkyle ou C₃-C₇-cycloalkyle;
R₂ signifie l'hydrogène, un groupe C₁-C₆-alkyle ou C₃-C₇-cycloalkyle et
R₃ signifie l'hydrogène ou un groupe C₁-C₆-alkyle, pour la préparation d'un agent pour l'administration systémique dans le sang des animaux domestiques pour la prévention de la propagation des puces sur lesdits animaux domestiques.

15. Utilisation d'un composé selon la revendication 14 de formule I, caractérisée en ce que la matière active est un des composés de formule I définis aux revendications 2 à 4.

16. Utilisation d'un composé selon la revendication 14 de formule I, caractérisée en ce qu'une quantité efficace de matière active est administrée à l'animal hôte avec la nourriture et qu'elle est absorbée par les puces se trouvant sur l'animal hôte avec le sang aspiré.

17. Un agent efficace du point de vue systémique pour la prévention d'une infestation par des puces chez des animaux domestiques, de préférence des chiens et des chats, caractérisé en ce qu'il contient dans la nourriture, une quantité efficace contre les puces d'un composé de formule I 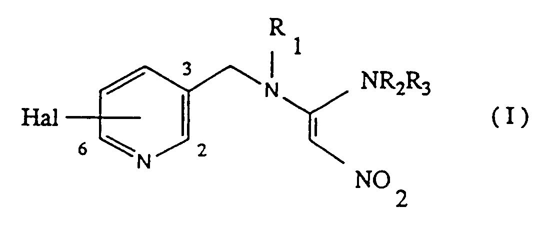
Hal signifie un halogène tel que le fluor, le chlore, le brome ou l'iode;
R₁ signifie l'hydrogène, un groupe C₁-C₆-alkyle ou C₃-C₇-cycloalkyle;
R₂ signifie l'hydrogène, un groupe C₁-C₆-alkyle ou C₃-C₇-cycloalkyle et
R₃ signifie l'hydrogène ou un groupe C₁-C₆-alkyle,
ensemble avec des excipients acceptables du point de vue physiologique par les animaux.

18. Un agent selon la revendication 17, caractérisé en ce qu'il s'agit de nourriture pour chiens ou pour chats.

19. Un agent selon la revendication 17, caractérisé en ce que la matière active est un composé de formule I selon l'une des revendications 2 à 4.

20. Un complément alimentaire pour la lutte systémique contre les puces chez des animaux domestiques, caractérisé en ce qu'il contient comme matière active, un composé de formule I 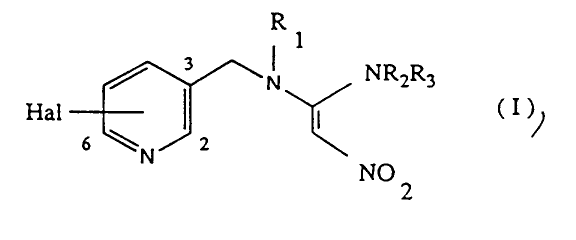 où
Hal signifie un halogène tel que le fluor, le chlore, le brome ou l'iode;
R₁ signifie l'hydrogène, un groupe C₁-C₆-alkyle ou C₃-C₇cycloalkyle;
R₂ signifie l'hydrogène, un groupe C₁-C₆-alkyle ou C₃-C₇cycloalkyle et
R₃ signifie l'hydrogène ou un groupe C₁-C₆-alkyle
en une quantité active contre les puces.

21. Un emballage contenant des doses unitaires d'un composé de formule I 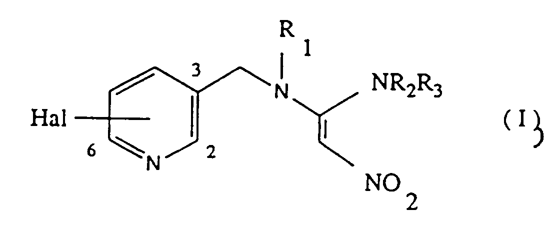 où
Hal signifie un halogène tel que le fluor, le chlore, le brome ou l'iode;
R₁ signifie l'hydrogène, un groupe C₁-C₆-alkyle ou C₃-C₇-cycloalkyle;
R₂ signifie l'hydrogène, un groupe C₁-C₆-alkyle ou C₃-C₇-cycloalkyle et
R₃ signifie l'hydrogène ou un groupe C₁-C₆-alkyle,
sous une forme destinée à l'utilisation par voie orale contre les puces chez les animaux domestiques, caractérisé en ce que la matière active se présente dans des doses unitaires prêtes à l'emploi comprises entre 0,01 mg/kg de poids corporel et environ 800 mg/kg de poids corporel.
